# EUROPEAN PATENT SPECIFICATION

(11) **EP 2 694 971 B1**
(45) Date of publication and mention of the grant of the patent: **04.03.2020**
(21) Application number: 12716825.0
(22) Date of filing: 30.03.2012
(51) Int. Cl.: G01N 33/574, G01N 33/74, G01N 33/543

(54) **STEROID RECEPTOR ASSAYS FOR DETECTING TUMOR CELLS**
STEROIDREZEPTORTESTS FÜR DEN NACHWEIS VON TUMORZELLEN
ANALYSES DE RÉCEPTEURS DE STÉROÏDES POUR LA DÉTECTION DE CELLULES CANCÉREUSES

(30) Priority: 01.04.2011 US 201161470618 P; 28.03.2012 US 201213432248
(43) Date of publication of application: 12.02.2014
(73) Proprietor: Menarini Silicon Biosystems S.p.A., 40013 Castel Maggiore (BO) (IT)
(72) Inventor: CHIANESE, David, A., Ardmore, PA 19003-2808 (US)
(74) Representative: Carpmaels & Ransford LLP
(86) International application number: PCT/US2012/031338
(87) International publication number: WO 2012/135560

(56) References cited:
- WO-A1-2011/002649
- WO-A2-2007/121459
- WO-A2-2012/006421
- PAVAO M ET AL: "Estrogen receptor antibodies: specificity and utility in detection, localization and analyses of estrogen receptor alpha and beta", STEROIDS, ELSEVIER SCIENCE PUBLISHERS, NEW YORK, NY, US, vol. 66, no. 1, 1 January 2001 (2001-01-01), pages 1-16, XP004220761, ISSN: 0039-128X, DOI: 10.1016/S0039-128X(00)00143-4 cited in the application
- DE BONO JOHANN S ET AL: "Potential applications for circulating tumor cells expressing the insulin-like growth factor-I receptor", CLINICAL CANCER RESEARCH, THE AMERICAN ASSOCIATION FOR CANCER RESEARCH, US, vol. 13, no. 12, 15 June 2007 (2007-06-15) , pages 3611-3616, XP002451690, ISSN: 1078-0432, DOI: 10.1158/1078-0432.CCR-07-0268
- Anonymous: "Product datasheet: Anti-Estrogen Receptor alpha antibody [TE111.5D11] ab16460", , 20 July 2017 (2017-07-20), XP055392760, Retrieved from the Internet: URL:http://www.abcam.com/Estrogen-Receptor -alpha-antibody-TE1115D11-ab16460.pdf [retrieved on 2017-07-20]
- Anthony Rhodes ET AL: "The Reliability of Rabbit Monoclonal Antibodies in the Immunohistochemical Assessment of Estrogen Receptors, Progesterone Receptors, and HER2 in Human Breast Carcinomas", AMERICAN JOURNAL OF CLINICAL PATHOLOGY, vol. 134, no. 4, 1 October 2010 (2010-10-01), pages 621-632, XP055392734, US ISSN: 0002-9173, DOI: 10.1309/AJCPOG3O3KTPZQNK
- Anonymous: "Product Datasheet: ER alpha/NR3A1 Antibody (E115), Cat # NB110-56961", , 20 July 2017 (2017-07-20), XP055392768, Retrieved from the Internet: URL:https://www.novusbio.com/PDFs/NB110-56 961.pdf [retrieved on 2017-07-20]
- "Monitoring expression of HER-2 on circulating epithelial cells in patients with advanced breast cancer", INTERNATIONAL JOURNAL OF ONCO, DEMETRIOS A. SPANDIDOS ED. & PUB, GR, vol. 21, 1 January 2002 (2002-01-01), pages 1111-1117, XP008083943, ISSN: 1019-6439

## Description

### FIELD OF THE INVENTION

The present invention relates to the fields of oncology and diagnostic testing, and more particularly to methods for cancer screening and for predicting and monitoring chemotherapy treatment responses, cancer recurrence or the like.

### BACKGROUND OF THE INVENTION

It is estimated that at there are at least several hundred thousand new cases of breast cancer every year. Such cases are currently confirmed by surgical means such as harvesting the tumor or the mastectomy tissue sectioning such tissue into paraffin blocks and using immunohistochemical methods to asses confirm the presence of steroid receptor proteins in such tissue. Due to the surgical procedure, such methods are not used for monitoring the progression of the disease state throughout the course of treatment. Throughout the years, these methods have been improved so that different characteristics of the tissue can be assessed, such as progesterone receptor, and HER/2-neu status, as well as confirming the presence of epitopes of the estrogen receptor. But in all cases this assessment requires a surgical procedure. Further, in the cases of patients whose breast tissue was removed early in their treatment, these methods are simply not available.

Circulating tumor cells ("CTCs") are present in the blood of patients with metastatic breast cancer and can be harvested throughout a patient's course of treatment.

These cells may be analyzed for their HER/2-neu status. Hayes et al. describes a method of analysing circulating tumor cells from patients with breast advanced cancer by quantifying the expression of HER-2 on CTCs (Monitoring expression of HER-2 on circulating epithelial cells in patients with advanced breast cancer, International Journal of ONCO, (2002) 21:1111-1117). Alternatively, as described in WO 2007/121459, an immunoassay is used to detect the expression of a steroid receptor from extracts of isolated carcinoma cells. However to date, there are no methods to determine the presence of epitopes of the N and C terminal regions of the estrogen receptor of such CTCs. These methods are found in the following invention.

### BRIEF DESCRIPTION OF THE DRAWINGS

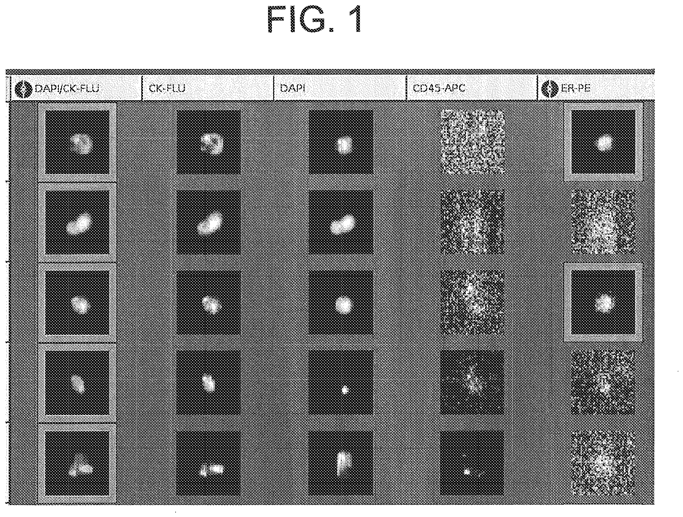

Figure 1 Images of patient samples showing CTCs positive for estrogen receptor α

### DETAILED DESCRIPTION OF THE INVENTION

The invention includes a method for characterizing circulating tumor cells in a biological sample from a patient with metastatic breast cancer, comprising the steps of:
a) contacting the biological sample with a protein which binds cell surface markers of circulating tumour cells and reacts specifically with circulating tumor cells, to the substantial exclusion of other sample components, and permits the separation of such bound circulating tumor cells from other sample components of the biological sample;
b) contacting the sample of step (a) with at least one reagent that specifically binds the sample of step (a), wherein the reagent is a substance that differentiates between different types of cells;
c) contacting the sample of step (b) with an agent having binding affinity for steroid receptors in cells; and
d) analyzing the sample to determine the presence of circulating tumor cells expressing steroid receptors;
wherein said method further comprises the step of determining the number of circulating tumour cells expressing the steroid receptors.

As used herein the term biological samples include but are not limited to whole blood, urine, serum, plasma, sputum, cerebrospinal fluid, amniotic fluid, lavage fluids and bone marrow. The preferred biological samples are whole blood, urine, plasma, sputum, and bone marrow, more preferably, whole blood, urine, plasma, sputum, most preferably whole blood.

The term "ligand" refers to proteins which bind cell surface markers of CTCs, which include but are not limited to adhesion molecules such as E cadherin, N cadherin, as well as monoclonal antibodies to EpCAM ,and the like. The preferred ligands include EpCAM antibodies. Further the ligand may comprise "magnetically responsive particles." Magnetically responsive particles are substances that facilitate magnetic separation. Typcally such particles are metallic or organometallic compositions. They may be optionally coated with a polymer, preferably a polymer of biological origin such as BSA. Complexes of the ligand and magnetically responsive particles may be bound to detectable labels which include but are not limited to fluorescent labels. A more detailed description of magnetically responsive particles is found in US. Pat. No. 5,985,153, entitled Magnetic Separation Apparatus and Mehtods Employing an Internal magnetic Capture Gradient and an External Transport Force,".

The term "other sample components" refers to components of the biological samples other than circulating tumor cells. Such components include but are not limited to white blood cells, normal red blood cells, leukocytes, endothelial cells, non-nucleated cells and the like.

The term "reagent" refers to a substance that differentiate between different types of cells. Examples of reagents include but are not limited to dyes such as DAPI, ethidium bromide and acridine orange and probes such as CD 45, CD41, PAC-1, CD4, CD8 CD56,CD146, CD34, CD38, cytokeratin and the like.

The term "agent" refers to antibodies to particular regions of the steroid receptor. If one is analyzing biological samples for CTCs from an estrogen positive breast cancer patient, antibodies to the N-terminal and C-terminal regions of the estrogen receptor are used. Antibodies to the N-terminal region include but are not limited to ID5, CF11, E115 (rb mono), SP-1 (rb mono), ER119.3 and the like. The preferred N-terminal region antibodies are ID5 and E115 (rb mono). Antibodies to the C-terminal region include but are not limited to H222, F10, TE111.5D11. The preferred antibodies to the C-terminal region H222. See M. Pavao et al. Estrogen receptor antibodies: specificity and utility in the detection, localization, and analyses of estrogen receptor α and β; Steroids 66 (2001) 1-16 for other monoclonal antibodies which are useful in this invention.

The term "steroid receptors" has its customary meaning. The preferred steroid receptors are the progesterone, testosterone, and estrogen receptors. The particularly preferred steroid receptors are all subtypes of the estrogen receptor, particularly estrogen receptor α.

Unless otherwise defined herein, scientific and technical terms used in connection with the present invention shall have the meanings that are commonly understood by those of ordinary skill in the art.

The isolation of circulating tumor cells from peripheral blood by immunomagnetic capture with magnetic particles is a multistep process that starts with stabilization and anticoagulation of the blood sample. Next immune magnetic capture and isolation of the target cells with a ferromagnetic particle conjugated to an epithelial specific monoclonal antibody is performed. Fluorescent labeling of the target cells and/or other characterization markers with dyes and fluorescent dye conjugated antibodies specific for the target rare event cell and antigens important to the biology of the cells are used to identify and characterize the cell populations. Finally, cells are visualized by magnetic localization in a cartridge with a window suitable for rare event identification and analysis by fluorescent microscopy, with software that measures the fluorescent signals generated from the microscopic analysis of the target rare event cells.

Further, the invention includes the use of a test kit for screening a patient sample for the presence of circulating tumor cells expressing a steroid receptor by performing the method of the invention, the test kit comprising:
a) a ligand that reacts specifically with circulating tumor cells, to the substantial exclusion of other sample components, and permits the separation of such bound circulating tumor cells from other sample components of the patient sample;
b) at least one reagent that specifically binds either or both circulating tumor cells and other sample components of the patient sample, wherein the reagent is a substance that differentiates between different types of cells; and
c) an agent having binding affinity for steroid receptors on the circulating tumor cells of the patient sample.
In order that this invention may be better understood, the following examples are set forth. These examples are for purposes of illustration only and are not to be construed as limiting the scope of the invention in any manner.

### EXAMPLE

Anticoagulation and Preservation of Blood Samples: The commercially available CellSave® tube was used to collect blood samples for this determination. This tube contains sodium EDTA, an anticoagulant, and blood preservative agents which stabilize the target cells in the sample enhancing the assay's ability to detect intact circulating tumor cells. This collection tube facilitates the shipping of samples to distant laboratories for analysis by stabilizing the cells at room temperature for periods of up to 96 hours. Published accounts detail the importance of the use of a preservative to prevent the fragmentation of circulating tumor cells seen in the peripheral blood of patients with solid tissue cancers such as adenocarcinoma of breast. These preservation methods are also compatible with the detection of estrogen receptor protein on these rare event cells.

Ferromagnetic Capture of Target Cells: Ferrofliud bound with an anti-EpCAM antibody clone VU-1D9 was used as part of the commercially available CellSearch® Epithelial Cell kit to localize circulating tumor cells from peripheral blood. This kit enriches rare event cells from as few as five cells in a 7.5 milliliter blood sample containing approximately 30,000,000 to 50,000,000 nucleated blood cells to a working volume of less than 0.5 milliliter with about 2,000 to 3000 leukocytes, and a resulting target cell enrichment of greater than 15,000 times. This reduced volume then allows for staining with fluorescent reagents to identify and characterize the captured rare event cells.

Staining of Captured Cells: The standard CellSearch® kit configuration contains a saponin-based reagent to permeabilize cell and nuclear membranes of intact cells, allowing the labeling of intra-cellular and nuclear proteins. The dye 4'6'diamidinophenylindole is used as an AT base pair, DNA specific dye that fluoresces in the UV range when bound to cell nuclei. This is used by the system to identify nucleated cells present in the isolated sample. Anti-cytokeratin monoclonal antibodies conjugated to fluorescent dyes such as phycoerytherin or fluorescein are used as epithelial specific markers to identify the rare event tumor cells in a population of blood elements. A fluorescent conjugate specific to CD45, a leukocute common antigen, was used to exclude contaminating white blood cells from the analysis.

Estrogen Receptor Specific Reagents: Commercially available monoclonal antibodies to both the N-terminal and C-terminal regions of the estrogen receptor alpha were sourced and conjugated to phycoerytherin and titrated against the cultured MCF-7 breast cancer cell line grown in estrogen depleted culture conditions. Antibody clones H222, SP-1, 1D5 and E115 were shown to work in both the model system of spiked MCF-7 cells in normal donor blood samples. These clones were also demonstrated to be positive in patient samples with advanced breast cancers, as determined on the CellSearch® system.

Analysis: All donor and patient samples were analyzed on the CellTracks® Analyzer II, an automated imaging system that scans cells localized in an analysis chamber as described in US. Pat No. 7,011,794, which is hereby incorporated by reference in its entirety. Images were processed and candidate circulating tumor cell images were presented for operator evaluation. All cells determined to be DAPI and cytokeratin positive, as well as CD45 negative. The cells identified as circulating tumor cells were then scored as being positive or negative for estrogen receptor or other steroid receptor, depending on the reagents used. Nuclear localization of the fluorescent signal was used as a morphologic criterion, matching the analysis used in immunohistochemistry in paraffin sections.
All of the commercial antibodies specific to estrogen receptor alpha evaluated in the model system were shown to be reactive in patient samples. A series of breast cancer patients was evaluated with the E115 rabbit monoclonal (Epitomics Inc.) conjugated to phycoerytherin as well as a fluorescein conjugated monoclonal to HER/2-neu tumor marker as defined by monoclonal antibody Her-81 from the University of Texas. Ten sample runs were performed with donor blood spiked with MCF-7 cells as an assay control. The control samples were all positive for CTC's, with an average of 78% +/- 16% of the spiked cells positive for estrogen receptor. The breast cancer patient data is summarized in table 1. Figure 1 is a gallery of typical CTC images from the CellTracks Analyzer II® obtained from patient samples

**Table 1. Summary of a Pilot Series of Breast Cancer Patients**

| **Breast Cancer Series n=28** | |
|---|---|
| 9 Patients Positive for CTC | 32% |
| 6 of 9 Samples ER pos | 67% |
| 3 of 9 HER2 pos | 33% |
| 3 of 9 ER / HER2 Dual Positives | 33% |

## Claims

1. A method for characterising circulating tumour cells in a biological sample obtained from a patient with metastatic breast cancer, comprising the steps of:
a) contacting the biological sample with a protein which binds cell surface markers of circulating tumour cells and reacts specifically with circulating tumour cells, to the substantial exclusion of other sample components, and permits the separation of such bound circulating tumour cells from other sample components of the biological sample;
b) contacting the sample of step (a) with at least one reagent that specifically binds the sample of step (a), wherein the reagent is a substance that differentiates between different types of cells;
c) contacting the sample of step (b) with an agent having binding affinity for steroid receptors in cells; and
d) analysing the sample to determine the presence of circulating tumour cells expressing steroid receptors;
wherein said method further comprises the step of determining the number of circulating tumour cells expressing the steroid receptors.

2. The method of claim 1, wherein the biological sample is blood or bone marrow.

3. The method of claim 1 or claim 2, wherein the agent that reacts specifically with circulating tumour cells binds to either or both N-terminal and C-terminal regions of said steroid receptors in said circulating tumour cells.

4. The method of claim 1 or claim 2, wherein said reagent is DAPI or the reagent binds to cytokeratin or CD45.

5. The method of claim 1 or claim 2, wherein the protein binds specifically to an epithelial cell adhesion epitope on the tumour cell.

6. The method of claim 1 or claim 2, wherein the protein further comprises magnetically responsive particles.

7. The method of claim 1 or claim 2, wherein the sample in step (d) is analysed by at least one process selected from the group consisting of multiparameter flow cytometry, immunofluorescent microscopy, laser scanning cytometry, bright field base image analysis, spectral imaging analysis, manual cell analysis, automated imaging system analysis, and automated cell analysis.

8. The method of claim 6 further comprising subjecting the product of step (a) to a magnetic field.

9. The method of claim 6, wherein the magnetically responsive particles are colloidal.

10. The method of claim 1 or claim 2, wherein the agent is anti-estrogen receptor antibody to the N-terminal region of the estrogen receptor and the anti-estrogen receptor antibody is selected from the group consisting of ID5, E115 (rb mono), ER119.3, and SP-1 (rb mono).

11. The method of claim 1 or claim 2, wherein the agent is anti-estrogen receptor antibody to the C-terminal region of the estrogen receptor and the anti-estrogen receptor antibody is selected from the group consisting of H222, F10, and TE111.5D11.

12. Use of a test kit for screening a patient sample for the presence of circulating tumour cells expressing a steroid receptor by performing the method of any one of claims 1-11, the test kit comprising:
a) a protein which binds cell surface markers of circulating tumour cells and reacts specifically with circulating tumour cells, to the substantial exclusion of other sample components, and permits the separation of such bound circulating tumour cells from other sample components of the patient sample;
b) at least one reagent that specifically binds either or both circulating tumour cells and other sample components of the patient sample, wherein the reagent is a substance that differentiates between different types of cells; and
c) an agent having binding affinity for steroid receptors on the circulating tumour cells of the patient sample.

## Patentansprüche

1. Verfahren zur Charakterisierung zirkulierender Tumorzellen in einer biologischen Probe, die von einer Patientin mit metastatischem Brustkrebs erhalten ist, umfassend die Schritte:
a) Inkontaktbringen der biologischen Probe mit einem Protein, das Zelloberflächenmarker zirkulierender Tumorzellen bindet und spezifisch mit zirkulierenden Tumorzellen reagiert, mit wesentlichem Ausschluss anderer Probenkomponenten, und die Abtrennung derartiger gebundener zirkulierender Tumorzellen von anderen Probenkomponenten der biologischen Probe ermöglicht;
b) Inkontaktbringen der Probe aus Schritt (a) mit wenigstens einem Reagens, das spezifisch die Probe aus Schritt (a) bindet, wobei das Reagens ein Stoff ist, der zwischen verschiedenen Typen von Zellen differenziert;
c) Inkontaktbringen der Probe aus Schritt (b) mit einem Mittel mit Bindungsaffinität für Steroidrezeptoren in Zellen; und
d) Analysieren der Probe zum Bestimmen des Vorhandenseins zirkulierender Tumorzellen, die Steroidrezeptoren exprimieren;
wobei das Verfahren ferner den Schritt des Bestimmens der Zahl von zirkulierenden Tumorzellen, die Steroidrezeptoren exprimierten, umfasst.

2. Verfahren gemäß Anspruch 1, wobei die biologische Probe Blut oder Knochenmark ist.

3. Verfahren gemäß Anspruch 1 oder Anspruch 2, wobei das Mittel, das spezifisch mit zirkulierenden Tumorzellen reagiert, an einen oder beide der N-terminalen und C-terminalen Bereiche der Steroidrezeptoren in den zirkulierenden Tumorzellen bindet.

4. Verfahren gemäß Anspruch 1 oder Anspruch 2, wobei das Reagens DAPI ist oder das Reagens an Zytokeratin oder CD45 bindet.

5. Verfahren gemäß Anspruch 1 oder Anspruch 2, wobei das Protein spezifisch an Epithelzellen-Adhäsionsepitop an der Tumorzelle bindet.

6. Verfahren gemäß Anspruch 1 oder Anspruch 2, wobei das Protein ferner magnetisch ansprechende Partikel umfasst.

7. Verfahren gemäß Anspruch 1 oder Anspruch 2, wobei die Probe bei Schritt (d) durch wenigstens ein Verfahren ausgewählt ist aus der Gruppe bestehend aus Multiparameter-Durchflusszytometrie, Immunfluoreszenzmikroskopie, Laserscanning-Zytometrie, Hellfeld-Bildanalyse, Spektralbildgebungsanalyse, manueller Zellanalyse, automatisierter Bildgebungssystemanalyse und automatisierter Zellanalyse analysiert wird.

8. Verfahren gemäß Anspruch 6, ferner umfassend Unterwerfen des Produkts aus Schritt (a) an ein Magnetfeld.

9. Verfahren gemäß Anspruch 6, wobei die magnetisch ansprechenden Partikel kolloidal sind.

10. Verfahren gemäß Anspruch 1 oder Anspruch 2, wobei das Mittel Anti-Estrogenrezeptor-Antikörper für den N-terminalen Bereich des Estrogenrezeptors ist und der Anti-Astrogenrezeptor-Antikörper ausgewählt ist aus der Gruppe bestehend aus ID5, E115 (rb mono), ER119.3 und SP-1 (rb mono).

11. Verfahren gemäß Anspruch 1 oder Anspruch 2, wobei das Mittel Anti-Estrogenrezeptor-Antikörper für den C-terminalen Bereich des Estrogenrezeptors ist und der Anti-Astrogenrezeptor-Antikörper ausgewählt ist aus der Gruppe bestehend aus H222, F10 und TE111.5D11.

12. Verwendung eines Testkits zum Screening einer Patientenprobe auf das Vorhandensein zirkulierender Tumorzellen, die einen Steroidrezeptor exprimieren, durch Durchführen des Verfahrens gemäß einem der Ansprüche 1-11, wobei das Testkit umfasst:
a) ein Protein, das Zelloberflächenmarker zirkulierender Tumorzellen bindet und spezifisch mit zirkulierenden Tumorzellen reagiert, mit wesentlichem Ausschluss anderer Probenkomponenten, und die Abtrennung derartiger gebundener zirkulierender Tumorzellen von anderen Probenkomponenten der Patientenprobe ermöglicht;
b) wenigstens ein Reagens, das eines oder beide von zirkulierenden Tumorzellen und anderen Probenkomponenten der Patientenprobe bindet, wobei das Reagens ein Stoff ist, der zwischen verschiedenen Typen von Zellen differenziert; und
c) ein Mittel mit Bindungsaffinität für Steroidrezeptoren an den zirkulierenden Tumorzellen der Patientenprobe.

## Revendications

1. Procédé pour caractériser des cellules tumorales circulantes dans un échantillon biologique obtenu d'un patient atteint d'un cancer du sein métastatique, comprenant les étapes consistant :
a) à mettre en contact l'échantillon biologique avec une protéine qui lie des marqueurs de surface de cellule de cellules tumorales circulantes et réagit spécifiquement avec des cellules tumorales circulantes, à l'exclusion importante d'autres composants d'échantillon, et permet la séparation de telles cellules tumorales circulantes liées d'autres composants d'échantillon de l'échantillon biologique ;
b) à mettre en contact l'échantillon de l'étape (a) avec au moins un réactif qui lie spécifiquement l'échantillon de l'étape (a), dans lequel le réactif est une substance qui fait une distinction entre différents types de cellules ;
c) à mettre en contact l'échantillon de l'étape (b) avec un agent ayant une affinité de liaison pour des récepteurs des stéroïdes dans des cellules ; et
d) à analyser l'échantillon pour déterminer la présence de cellules tumorales circulantes exprimant des récepteurs des stéroïdes ;
dans lequel ledit procédé comprend en outre l'étape consistant à déterminer le nombre de cellules tumorales circulantes exprimant les récepteurs des stéroïdes.

2. Procédé selon la revendication 1, dans lequel l'échantillon biologique est du sang ou de la moelle osseuse.

3. Procédé selon la revendication 1 ou la revendication 2, dans lequel l'agent qui réagit spécifiquement avec des cellules tumorales circulantes, se lie à l'une ou l'autre ou à l'une et l'autre des régions N-terminale et C-terminale desdits récepteurs des stéroïdes dans lesdites cellules tumorales circulantes.

4. Procédé selon la revendication 1 ou la revendication 2, dans lequel ledit réactif est le DAPI ou le réactif se lie à la cytokératine ou à la CD45.

5. Procédé selon la revendication 1 ou la revendication 2, dans lequel la protéine se lie spécifiquement à un épitope d'adhérence des cellules épithéliales sur la cellule tumorale.

6. Procédé selon la revendication 1 ou la revendication 2, dans lequel la protéine comprend en outre des particules magnétiquement sensibles.

7. Procédé selon la revendication 1 ou la revendication 2, dans lequel l'échantillon à l'étape (d) est analysé par au moins un processus sélectionné dans le groupe constitué par une cytométrie en flux multiparamétrique, une microscopie par immunofluorescence, une cytométrie à balayage laser, une analyse d'image de base en champ clair, une analyse d'imagerie spectrale, une analyse de cellule manuelle, une analyse de système d'imagerie automatisée et une analyse de cellule automatisée.

8. Procédé selon la revendication 6, consistant en outre à soumettre le produit de l'étape (a) à un champ magnétique.

9. Procédé selon la revendication 6, dans lequel les particules magnétiquement sensibles sont colloïdales.

10. Procédé selon la revendication 1 ou la revendication 2, dans lequel l'agent est un anticorps anti-récepteur aux œstrogènes pour la région N-terminale du récepteur d'œstrogènes et l'anticorps anti-récepteur aux œstrogènes est sélectionné dans le groupe constitué par l'ID5, l'E115 (rb mono), l'ER119.3 et le SP-1 (rb mono).

11. Procédé selon la revendication 1 ou la revendication 2, dans lequel l'agent est un anticorps anti-récepteur aux œstrogènes pour la région C-terminale du récepteur d'œstrogènes et l'anticorps anti-récepteur aux œstrogènes est sélectionné dans le groupe constitué par le H222, le F10 et le TE111.5D11.

12. Utilisation d'un kit de test de criblage d'un échantillon de patient pour la présence de cellules tumorales circulantes exprimant un récepteur des stéroïdes en réalisant le procédé selon l'une quelconque des revendications 1 à 11, le kit de test comprenant :
a) une protéine qui lie des marqueurs de surface de cellule de cellules tumorales circulantes et réagit spécifiquement avec des cellules tumorales circulantes, à l'exclusion importante d'autres composants d'échantillon, et permet la séparation de telles cellules tumorales circulantes liées d'autres composants d'échantillon de l'échantillon du patient ;
b) au moins un réactif qui lie spécifiquement des cellules tumorales circulantes et/ou d'autres composants d'échantillon de l'échantillon du patient, dans lequel le réactif est une substance qui fait une distinction entre différents types de cellules ; et
c) un agent ayant une affinité de liaison pour des récepteurs des stéroïdes sur les cellules tumorales circulantes de l'échantillon du patient.
